# EUROPÄISCHE PATENTANMELDUNG

(11) **EP 2 617 407 A2**
(43) Veröffentlichungstag der Anmeldung: **24.07.2013**
(21) Anmeldenummer: 12196422.5
(22) Anmeldetag: 11.12.2012
(51) Int. Cl.: A61K 8/34, A61Q 1/14, A61K 8/67, A61K 8/73, A61K 8/90, A61K 8/97

(54) **Mildes Gesichtsreinigungsmittel mit Haut pflegenden Eigenschaften**

(30) Priorität: 22.12.2011 DE 102011089576
(71) Anmelder: Henkel AG&Co. KGAA, 40589 Düsseldorf (DE)
(72) Erfinder: Mewes, Karsten Rüdiger, 55128 Mainz (DE); Schelges, Heike, 47877 Willich (DE); Hamacher, Birgit, 41470 Neuss (DE)

(57) **Zusammenfassung**

Gegenstand der vorliegenden Anmeldung sind milde Reinigungszusammensetzungen für das Gesicht zur Entfernung von Make-up, insbesondere im Augenbereich, die ausgewählte pflegende Inhaltsstoffe enthalten.

## Beschreibung

Gegenstand der vorliegenden Anmeldung sind milde Reinigungszusammensetzungen für das Gesicht zur Entfernung von Make-up, insbesondere im Augenbereich, die ausgewählte pflegende Inhaltsstoffe enthalten.

Die Formulierung von Make-up-Entfernern ist für den Fachmann immer eine Herausforderung. Fett- und pigmenthaltige Make-up-Reste erfordern für ihre vollständige Entfernung von der Haut eigentlich ein hoch wirksames tensidisches Reinigungsmittel. Andererseits können solche Mittel die Haut zu stark entfetten und sie so empfindlicher und angreifbarer gegenüber Stressfaktoren machen. Da das Make-up besonders häufig im Augenbereich aufgetragen wird, der auf Tenside besonders sensibel reagiert, sollte ein Make-up-Entferner, der insbesondere für den Augenbereich geeignet sein soll, aber eigentlich möglichst wenig Tenside enthalten.

Nach der Anwendung von üblichen Make-up-Entfernern für den Augenbereich fühlt sich die Haut um die Augen herum oft trocken und gespannt an. Gerade diese Hautpartien sind sehr empfindlich, da dünn im Aufbau, und neigen zu Rötungen und längerfristig zu Faltenbildung.

Der vorliegenden Anmeldung lag die Aufgabe zu Grunde, einen Make-up-Entferner insbesondere für den Augenbereich zu entwickeln, der sowohl gut abschminkt als auch die Haut schont bzw. pflegt.

Es wurde festgestellt, dass ausgewählte kosmetische Wirkstoffe besonders gut in der Lage sind, die nachteiligen Wirkungen der reinigenden Bestandteile der tensidischen Bestandteile eines Make-up-Entferners in Bezug auf Hautentfettung und Hautreizung auszugleichen.

Gegenstand der vorliegenden Anmeldung ist daher eine milde Reinigungszusammensetzung zur Entfernung von Make-up, insbesondere im Augenbereich, enthaltend - jeweils bezogen auf das Gesamtgewicht der Reinigungszusammensetzung -
a) 60 - 90 Gew.-% Wasser,
b) in einer Gesamtmenge von 1 - 12 Gew.-% mindestens ein C2-C9-Polyol,
c) in einer Gesamtmenge von 1 - 5 Gew.-% mindestens ein Poloxamer,
d) in einer Gesamtmenge von 0,05 - 0,5 Gew.-% mindestens ein Chitosan-Salz,
e) in einer Gesamtmenge von 0,01 - 5 Gew.-% mindestens einen kosmetischen Wirkstoff, ausgewählt aus:
   e.i) Glycosaminoglycanen, insbesondere Hyaluronsäure und Derivaten der Hyaluronsäure, und/oder
   e.ii) Aktivatoren der Hyaluronsäuresynthese in der Haut, und/oder
   e.iii) Vitaminen sowie Estern, Ethern und Salzen hiervon, und/oder
   e.iv) Antioxidantien, und/oder
   e.v) Extrakten aus Pflanzen und/oder Hefen, und/oder
   e.vi) sowie Mischungen dieser Wirkstoffe.

Ein weiterer Gegenstand der vorliegenden Anmeldung ist die Verwendung einer milden Reinigungszusammensetzung, enthaltend - jeweils bezogen auf das Gesamtgewicht der Reinigungszusammensetzung -
a) 60 - 90 Gew.-% Wasser,
b) in einer Gesamtmenge von 1 - 12 Gew.-% mindestens ein C2-C9-Polyol,
c) in einer Gesamtmenge von 1 - 5 Gew.-% mindestens ein Poloxamer,
d) in einer Gesamtmenge von 0,05 - 0,5 Gew.-% mindestens ein Chitosan-Salz,
e) in einer Gesamtmenge von 0,01 - 5 Gew.-% mindestens einen kosmetischen Wirkstoff, ausgewählt aus:
   e.i) Glycosaminoglycanen, insbesondere Hyaluronsäure und Derivaten der Hyaluronsäure, und/oder
   e.ii) Aktivatoren der Hyaluronsäuresynthese in der Haut, und/oder
   e.iii) Vitaminen sowie Estern, Ethern und Salzen hiervon, und/oder
   e.iv) Antioxidantien, und/oder
   e.v) Extrakten aus Pflanzen und/oder Hefen, und/oder
   e.vi) sowie Mischungen dieser Wirkstoffe,
   zur Entfernung von Make-up, insbesondere im Augenbereich.

Die erfindungsgemäßen Reinigungszusammensetzungen enthalten, jeweils bezogen auf ihr Gesamtgewicht, insgesamt 60 - 90 Gew.-%, bevorzugt 70 - 88 Gew.-% und besonders bevorzugt 75 - 84 Gew.-% Wasser.

Als zweiten obligatorischen Inhaltsstoff enthalten die erfindungsgemäßen Reinigungszusammensetzungen, jeweils bezogen auf ihr Gesamtgewicht, in einer Gesamtmenge von 1 - 12 Gew.-%, bevorzugt 2 - 11 Gew.-%, besonders bevorzugt 4 - 10 Gew.-%, mindestens ein C2-C9-Polyol. Erfindungsgemäß bevorzugte C2-C9-Polyole sind ausgewählt aus 1,2-Propylenglycol, Glycerin, 2-Methyl-1,3-propandiol, 1,2-Butylenglycol, 1,3-Butylenglycol, 1,4-Butylenglycol, Pentylenglycolen wie 1,2-Pentandiol und 1,5-Pentandiol, Hexandiolen wie 1,2-Hexandiol und 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit, cis-1,4-Dimethylolcyclohexan, trans-1,4-Dimethylolcyclohexan, beliebigen Isomeren-Gemischen von cis- und trans-1,4-Dimethylolcyclohexan, sowie Mischungen der vorgenannten Substanzen.

Besonders bevorzugt ist Dipropylenglycol, insbesondere in einer Menge von 4 - 10 Gew.-%, bezogen auf das Gesamtgewicht der Reinigungszusammensetzung.

Als dritten obligatorischen Inhaltsstoff enthalten die erfindungsgemäßen Reinigungszusammensetzungen, jeweils bezogen auf ihr Gesamtgewicht, in einer Gesamtmenge von 1 - 5 Gew.-%, bevorzugt 2 - 4 Gew.-%, besonders bevorzugt 3 - 3,5 Gew.-%, mindestens ein Poloxamer. Besonders bevorzugt ist Poloxamer 184, besonders bevorzugt in einer Menge von2 - 4 Gew.-%, besonders bevorzugt 3 - 3,5 Gew.-%, jeweils bezogen auf das Gesamtgewicht der Reinigungszusammensetzung.

Als vierten obligatorischen Inhaltsstoff enthalten die erfindungsgemäßen Reinigungszusammensetzungen, jeweils bezogen auf ihr Gesamtgewicht, in einer Gesamtmenge von 0,05 - 0,5 Gew.-%, bevorzugt 0,1 - 0,3 Gew.-%, mindestens ein Chitosan-Salz. Chitosan selbst ist wasserunlöslich, seine Salze mit Säuren, insbesondere mit organischen Säuren, sind jedoch hervorragend wasserlöslich und unterstützen die Reinigungsleistung der erfindungsgemäßen Reinigungszusammensetzungen. Besonders bevorzugt sind Chitosanglycolat und Chitosanlactat.

In einer weiteren bevorzugten Ausführungsform der Erfindung ist das mindestens eine Glycosaminoglycan ausgewählt aus Hyaluronsäure und Estern, Ethern und Salzen der Hyaluronsäure, insbesondere Natriumhyaluronatdimethylsilanol, Natriumhyaluronat und Dimethylsilanolhyaluronat, weiterhin Dextran, Dextransulfat, Chondroitin-4-sulfat und Chondroitin-6-sulfat, sowie beliebigen Mischungen dieser Substanzen. Glycosaminoglycane sind hoch wirksam, daher genügen niedrige Konzentrationen. Bevorzugte erfindungsgemäße Reinigungszusammensetzungen enthalten, jeweils bezogen auf ihr Gesamtgewicht, in einer Gesamtmenge von 0,001 - 0,2 Gew.-%, bevorzugt 0,01 - 0,1 Gew.-%, besonders bevorzugt 0,03 - 0,08 Gew.-%, mindestens ein Glycosaminoglycan, das bevorzugt ausgewählt ist aus Natriumhyaluronatdimethylsilanol, Natriumhyaluronat und Dimethylsilanolhyaluronat sowie Mischungen hiervon.

In einer weiteren bevorzugten Ausführungsform der Erfindung ist der mindestens eine Aktivator der Hyaluronsäuresynthese in der Haut ausgewählt aus Dipeptiden, insbesondere aus Prolin-Hydroxyprolin, bevorzugt in einer Gesamtmenge von 1-1000 nmol/g, besonders bevorzugt 100 - 500 nmol/g.

In einer weiteren bevorzugten Ausführungsform der Erfindung ist die mindestens eine Substanz, die ausgewählt ist aus Vitaminen, ihren Estern, Ethern und/oder Salzen, ausgewählt aus Ascorbinsäure, Ascorbinsäureestern, Ascorbinsäureethern und den Salzen dieser Verbindungen, Retinol und den Estern des Retinols sowie Tocopherol und den Tocopherylestern.

Vitamin C (Ascorbinsäure) wird bevorzugt in Mengen von 0,1 bis 3 Gew.-%, bezogen auf die gesamte Zusammensetzung, eingesetzt. Die Verwendung der Derivate Ascorbylpalmitat, -stearat, - dipalmitat, -acetat, Mg-Ascorbylphosphat, Na-Ascorbylphosphat, Natrium- und Magnesiumascorbat, Dinatriumascorbylphosphat und -sulfat, Kaliumascorbyltocopherylphosphat, Chitosanascorbat oder Ascorbylglucosid kann erfindungsgemäß bevorzugt sein. Die Verwendung in Kombination mit Tocopherolen kann ebenfalls erfindungsgemäß bevorzugt sein.

Zur Vitamin E-Gruppe zählen Tocopherol, insbesondere a-Tocopherol, und seine Derivate. Bevorzugte Derivate sind insbesondere die Ester, wie Tocopherylacetat, -nicotinat, -phosphat, - succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in Mengen von 0,05 - 1 Gew.-%, bezogen auf die gesamte Zusammensetzung, enthalten.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester, wie insbesondere Retinylpalmitat und Retinylacetat, in Betracht. Die erfindungsgemäßen Zusammensetzungen enthalten die Vitamin A-Komponente bevorzugt in einer Gesamtmenge von 0,05 - 1 Gew.-%, bezogen auf die gesamte Zusammensetzung.

In einer weiteren bevorzugten Ausführungsform der Erfindung ist das mindestens eine Antioxidans ausgewählt aus Chromanen, Chromenen, Carotinoiden, Flavonoiden, Isoflavonoiden, Phenolen, Polyphenolen, Hydrochinonen und Biochinonen.

Ein bevorzugtes Antioxidans ist ausgewählt aus mindestens einem 6,7-disubstituierten 2,2-Dialkylchroman oder -chromen der allgemeinen Formel (I) oder (II), 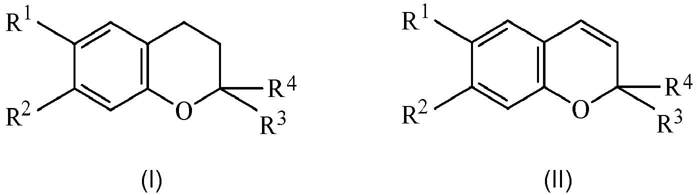 wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen. Besonders bevorzugt ist 2,2-Dimethyl-6-hydroxy-7-methoxy-chroman mit der systematischen Bezeichnung 3,4-Dihydro-7-methoxy-2,2-dimethyl-2H-1-benzopyran-6-ol und der INCI-Bezeichnung Dimethylmethoxy Chromanol. Die Substanz ist unter dem Handelsnamen Lipochroman-6 von der Firma Lipotec S.A. erhältlich.

Bevorzugt ist 3,4-Dihydro-7-methoxy-2,2-dimethyl-2H-1-benzopyran-6-ol in einer Gesamtmenge von 0,001 - 2 Gew.-%, bevorzugt 0,005 - 1 Gew.-% und besonders bevorzugt 0,01 - 0,1 Gew.-%, jeweils bezogen auf die gesamte Zusammensetzung, enthalten.

Ein weiteres bevorzugtes Antioxidans ist Coenzym Q10, ein Biochinon, das bevorzugt in einer Menge von 0,0001 bis 1 Gew.-%, bevorzugt 0,001 bis 0,5 Gew.-% und besonders bevorzugt 0,005 bis 0,1 Gew.-%, jeweils bezogen auf die gesamte kosmetische Zusammensetzung, enthalten ist.

In einer weiteren bevorzugten Ausführungsform der Erfindung ist der mindestens eine Extrakt aus Pflanzen und/oder Hefen ausgewählt aus den wasserlöslichen Extrakten von Aloe vera und Saccaromyces cerevisiae. Der Extrakt oder die Extrakte sind bevorzugt in einer Gesamtmenge von 0,0001 bis 1 Gew.-%, bevorzugt 0,001 bis 0,5 Gew.-% und besonders bevorzugt 0,005 bis 0,1 Gew.-%, jeweils bezogen auf die gesamte kosmetische Zusammensetzung, enthalten.

Um die hervorragenden Pflegeeigenschaften und die milde, aber sehr gute Reinigungsleistung der erfindungsgemäßen Reinigungszusammensetzungen nicht zu beeinträchtigen, sind Art und Menge weiterer optionaler Zusatzstoffe sehr begrenzt. Bevorzugte optionale Zusatzstoffe sind Parfüm, Konservierungsmittel, Komplexbildner, 0,01 - 0,1 Gew.-% an wasserlöslichem UV-Filter als Produktschutz sowie Säurepuffer wie Milchsäure/Lactat oder Citronensäure/Citrat, um die Reinigungszusammensetzungen auf einen bevorzugten pH-Wert von 4,7 bis 5,2 einzustellen. Weiterhin kann optional mindestens ein kosmetisches Öl in einer Gesamtmenge von 0,05 bis 0,2 Gew.-% enthalten sein. Weiterhin kann optional mindestens ein Solubilisierungsmittel in einer Gesamtmenge von 0,05 bis 1,2 Gew.-%, bezogen auf die gesamte Zusammensetzung, enthalten sein. Die optionalen Zusatzstoffe sind bevorzugt in einer Gesamtmenge von 0,05 bis 3,5 Gew.-%, bezogen auf die gesamte Zusammensetzung, enthalten.

Erfindungsgemäße Beispiele

| **Ingredients (EU)** | **Gew.-%-Anteil** |
|---|---|
| Dipropylene Glycol | 10 |
| Poloxamer 184 | 2,2 |
| PEG-60 Hydrogenated Castor Oil | 0,5 |
| 1,2-Propylene Glycol | 1,5 |
| Parfum | 0,2 |
| Chitosan Lactate | 0,2 |
| Lactic Acid | 0,12 |
| Oenothera Biennis Oil | 0,1 |
| Aloe vera Extract | 0,1 |
| Tetrasodium EDTA | 0,11 |
| Benzophenone-4 | 0,05 |
| Phenoxyethanol | 0,5 |
| Sodium Benzoate | 0,4 |
| Methylparaben | 0,02 |
| Ethylparaben | 0,01 |
| Aqua | ad 100 |

| **Ingredients (EU)** | **Gew.-%-Antell** |
|---|---|
| Dipropylene Glycol | 10 |
| Poloxamer 184 | 2,2 |
| PEG-60 Hydrogenated Castor Oil | 0,5 |
| 1,2-Propylene Glycol | 1,5 |
| Parfum | 0,2 |
| Chitosan Lactate | 0,2 |
| Lactic Acid | 0,12 |
| Oenothera Biennis Oil | 0,1 |
| Dimethylsilanolhyaluronat | 0,01 |
| Tetrasodium EDTA | 0,11 |
| Benzophenone-4 | 0,05 |
| Phenoxyethanol | 0,5 |
| Sodium Benzoate | 0,4 |
| Methylparaben | 0,02 |
| Ethylparaben | 0,01 |
| Aqua | ad 100 |

| **Ingredients (EU)** | **Gew.-%-Anteil** |
|---|---|
| Dipropylene Glycol | 10 |
| Poloxamer 184 | 2,2 |
| PEG-60 Hydrogenated Castor Oil | 0,5 |
| 1,2-Propylene Glycol | 1,5 |
| Parfum | 0,2 |
| Chitosan Lactate | 0,2 |
| Lactic Acid | 0,12 |
| Magnesiumascorbylphosphat | 0,05 |
| Dimethylsilanolhyaluronat | 0,01 |
| Tetrasodium EDTA | 0,11 |
| Benzophenone-4 | 0,05 |
| Phenoxyethanol | 0,5 |
| Sodium Benzoate | 0,4 |
| Methylparaben | 0,02 |
| Ethylparaben | 0,01 |
| Aqua | ad 100 |

| **Ingredients (EU)** | **Gew.-%-Antell** |
|---|---|
| Dipropylene Glycol | 10 |
| Poloxamer 184 | 2,2 |
| PEG-60 Hydrogenated Castor Oil | 0,5 |
| 1,2-Propylene Glycol | 1,5 |
| Parfum | 0,2 |
| Chitosan Lactate | 0,2 |
| Lactic Acid | 0,12 |
| Magnesiumascorbylphosphat | 0,05 |
| Retinylpalmitat | 0,05 |
| Tetrasodium EDTA | 0,11 |
| Benzophenone-4 | 0,05 |
| Phenoxyethanol | 0,5 |
| Sodium Benzoate | 0,4 |
| Methylparaben | 0,02 |
| Ethylparaben | 0,01 |
| Aqua | ad 100 |

| **Ingredients (EU)** | **Gew.-%-Anteil** |
|---|---|
| Dipropylene Glycol | 10 |
| Poloxamer 184 | 2,2 |
| PEG-60 Hydrogenated Castor Oil | 0,5 |
| 1,2-Propylene Glycol | 1,5 |
| Parfum | 0,2 |
| Chitosan Lactate | 0,2 |
| Lactic Acid | 0,12 |
| Saccharomyces cerevisiae Extract | 0,1 |
| Dimethylsilanolhyaluronat | 0,01 |
| Tetrasodium EDTA | 0,11 |
| Benzophenone-4 | 0,05 |
| Phenoxyethanol | 0,5 |
| Sodium Benzoate | 0,4 |
| Methylparaben | 0,02 |
| Ethylparaben | 0,01 |
| Aqua | ad 100 |

| **Ingredients (EU)** | **Gew.-%-Anteil** |
|---|---|
| Dipropylene Glycol | 10 |
| Poloxamer 184 | 2,2 |
| PEG-60 Hydrogenated Castor Oil | 0,5 |
| 1,2-Propylene Glycol | 1,5 |
| Parfum | 0,2 |
| Chitosan Lactate | 0,2 |
| Lactic Acid | 0,12 |
| Tocopherylacetat | 0,05 |
| Dimethylsilanolhyaluronat | 0,01 |
| Tetrasodium EDTA | 0,11 |
| Benzophenone-4 | 0,05 |
| Phenoxyethanol | 0,5 |
| Sodium Benzoate | 0,4 |
| Methylparaben | 0,02 |
| Ethylparaben | 0,01 |
| Aqua | ad 100 |

## Patentansprüche

1. Milde Reinigungszusammensetzung zur Entfernung von Make-up, insbesondere im Augenbereich, enthaltend - jeweils bezogen auf das Gesamtgewicht der Reinigungszusammensetzung -
a) 60 - 90 Gew.-% Wasser,
b) in einer Gesamtmenge von 1 - 12 Gew.-% mindestens ein C2-C9-Polyol,
c) in einer Gesamtmenge von 1 - 5 Gew.-% mindestens ein Poloxamer,
d) in einer Gesamtmenge von 0,05 - 0,5 Gew.-% mindestens ein Chitosan-Salz,
e) in einer Gesamtmenge von 0,01 - 5 Gew.-% mindestens einen kosmetischen Wirkstoff, ausgewählt aus:
e.i) Glycosaminoglycanen, insbesondere Hyaluronsäure und Derivaten der Hyaluronsäure, und/oder
e.ii) Aktivatoren der Hyaluronsäuresynthese in der Haut, und/oder
e.iii) Vitaminen sowie Estern, Ethern und Salzen hiervon, und/oder
e.iv) Antioxidantien, und/oder
e.v) Extrakten aus Pflanzen und/oder Hefen, und/oder
e.vi) sowie Mischungen dieser Wirkstoffe.

2. Reinigungszusammensetzung nach Anspruch 1, **dadurch gekennzeichnet, dass** das mindestens eine Glycosaminoglycan ausgewählt ist aus Hyaluronsäure und Estern, Ethern und Salzen der Hyaluronsäure, weiterhin Dextran, Dextransulfat, Chondroitin-4-sulfat und Chondroitin-6-sulfat, sowie beliebigen Mischungen dieser Substanzen.

3. Reinigungszusammensetzung nach Anspruch 1 oder 2, **dadurch gekennzeichnet, dass** die Aktivatoren der Hyaluronsäuresynthese in der Haut ausgewählt sind aus Dipeptiden, insbesondere aus Prolin-Hydroxyprolin.

4. Reinigungszusammensetzung nach Anspruch 1, 2 oder 3, **dadurch gekennzeichnet, dass** die Vitamine, ihre Ester, Ether und/oder Salze ausgewählt sind aus Ascorbinsäure, Ascorbinsäureestern, Ascorbinsäureethern und den Salzen dieser Verbindungen, Retinol und den Estern des Retinols sowie Tocopherol und den Tocopherylestern.

5. Reinigungszusammensetzung nach Anspruch 1, 2, 3 oder 4, **dadurch gekennzeichnet, dass** die Antioxidantien ausgewählt sind aus Chromanen, Chromenen, Carotinoiden, Flavonoiden, Isoflavonoiden, Phenolen, Polyphenolen, Hydrochinonen und Biochinonen.

6. Reinigungszusammensetzung nach Anspruch 1, 2, 3, 4 oder 5, **dadurch gekennzeichnet, dass** die Extrakte aus Pflanzen und/oder Hefen ausgewählt sind aus den wasserlöslichen Extrakten von Aloe vera und Saccaromyces cerevisiae.

7. Reinigungszusammensetzung nach Anspruch 1, 2, 3, 4, 5 oder 6, **dadurch gekennzeichnet, dass** in einer Gesamtmenge von 0,05 bis 3,5 Gew.-% Zusatzstoffe, ausgewählt aus Parfüm, Konservierungsmitteln, Komplexbildnern, 0,01 - 0,1 Gew.-% an wasserlöslichem(n) UV-Filter(n), Säurepuffern wie Milchsäure/Lactat oder Citronensäure/Citrat, mindestens einem kosmetischen Öl in einer Gesamtmenge von 0,05 bis 0,2 Gew.-%, mindestens einem weiteren Solubilisierungsmittel in einer Gesamtmenge von 0,05 bis 1,2 Gew.-%, jeweils bezogen auf die gesamte Zusammensetzung, enthalten sind.

8. Reinigungszusammensetzung nach Anspruch 1, 2, 3, 4, 5, 6 oder 7, **gekennzeichnet durch** einen pH-Wert bei 20°C von 4,7 - 5,2.

9. Verwendung einer milden Reinigungszusammensetzung, enthaltend -jeweils bezogen auf das Gesamtgewicht der Reinigungszusammensetzung:
a) 60 - 90 Gew.-% Wasser,
b) in einer Gesamtmenge von 1 - 12 Gew.-% mindestens ein C2-C9-Polyol,
c) in einer Gesamtmenge von 1 - 5 Gew.-% mindestens ein Poloxamer,
d) in einer Gesamtmenge von 0,05 - 0,5 Gew.-% mindestens ein Chitosan-Salz,
e) in einer Gesamtmenge von 0,01 - 5 Gew.-% mindestens einen kosmetischen Wirkstoff, ausgewählt aus:
e.i) Glycosaminoglycanen, insbesondere Hyaluronsäure und Derivaten der Hyaluronsäure, und/oder
e.ii) Aktivatoren der Hyaluronsäuresynthese in der Haut, und/oder
e.iii) Vitaminen sowie Estern, Ethern und Salzen hiervon, und/oder
e.iv) Antioxidantien, und/oder
e.v) Extrakten aus Pflanzen und/oder Hefen, und/oder
e.vi) sowie Mischungen dieser Wirkstoffe,
zur Entfernung von Make-up, insbesondere im Augenbereich.
